# EUROPEAN PATENT SPECIFICATION

(11) **EP 0 445 193 B1**
(45) Date of publication and mention of the grant of the patent: **01.02.1995**
(21) Application number: 90900032.5
(22) Date of filing: 28.11.1989
(51) Int. Cl.: B65D 39/12, B65D 43/10

(54) **Closure for containers**
Behälterverschluss
Opercule pour récipients

(30) Priority: 28.11.1988 AU 1686/88
(43) Date of publication of application: 11.09.1991
(73) Proprietor: GOODALL, Donald Terry, Blakehurst, N.S.W. 2220 (AU)
(72) Inventor: GOODALL, Donald Terry, Blakehurst, N.S.W. 2220 (AU)
(74) Representative: Jenkins, Peter David
(86) International application number: AU8900515
(87) International publication number: WO9006266

(56) References cited:
- AU-A- 956 327
- AU-A- 1 888 283
- AU-A- 3 007 471
- AU-A- 3 034 971
- AU-A- 5 784 769
- AU-A- 6 557 986
- AU-A- 8 685 782
- AU-B- 41 538
- AU-B- 170 838
- AU-B- 2 470 962
- AU-B- 2 745 867
- AU-B- 3 144 384
- AU-B- 3 467 684
- AU-B- 4 110 178
- AU-B- 5 172 164
- AU-B- 5 291 973
- AU-B- 7 393 574
- FR-A- 1 218 702
- GB-A- 1 080 005
- US-A- 1 732 834
- US-A- 3 028 985
- US-A- 3 279 643

## Description

This invention relates to a removable closure for containers.

It has been discovered that a tubular body having an internal or external annular ring undergoes distortion when the direction of the annular ring is changed. In particular this distortion is in the form of an annular lip formed on the wall of the tube opposite the joint of the ring and tubular body. Thus a tubular body having an internal annular ring will form a radially outwardly extending annular lip while an external ring will cause a radially inwardly extending annular lip.

Many types of closures for containers have been developed over the years including those made of elastomeric material.

GB-1,080,005 dicsloses a closure for bottles and like containers comprising a plug like member dimensioned to fit within the neck of a container. It includes a cap with an integral skirt adapted to fit with the outlet of a container and engage the side walls. The cap includes a number of concentric grooves which permit the cap to flex between two positions.

In one position the cap can be slidably inserted within the container outlet and in another position the cap is moved with the aid of the concentric grooves to apply radial force to the skirt whereby the skirt is locked adjacent the inner wall of the container throughout its length.

The present invention relates to a closure for a container which can be sealed against the outer or inner wall or both of a container outlet and which effectively locks and seals the container outlet.

Accordingly, the present invention provides a removable closure for a container having a cylindrical container wall with an inner and an outer surface defining an opening, said closure comprising a lid for cooperating with the cylindrical container wall to close said opening, said lid comprising a disc portion extending across said opening and a cylindrical tubular wall extending along the outer surface of said cylindrical container wall in closely spaced relation when said lid is mounted telescopically on said container characterised in that said tubular wall has a lower end with a frusto-conical wall portion extending therefrom circumferentially about the periphery of said tubular wall, said frusto-conical wall portion being displaced between either of two stable states, one of said stable states being defined by said frusto-conical wall portion converging towards the lower end of the tubular wall thereby presenting a smooth inner peripheral surface to said cylindrical container wall and permitting said lid to be slidably mounted and dismounted from said container, and the other of said stable states being defined by said frusto-conical wall portion diverging away from the lower end of the tubular wall thereby deforming said outer tubular wall adjacent said lower end to create an inwardly facing peripheral locking bead which, when the lid is mounted on the container, engages and grips said outer surface of said cylindrical container wall thereby preventing removal of the lid from the container.

Preferably, the lid further comprises an inner tubular wall engageable within the container opening, said disc portion comprising a frusto-conical annular portion connected to said inner tubular wall at a lower end of the inner tubular wall, said frusto-conical annular portion being displaceable between either of two stable states, one of said stable states being defined by said frusto-conical annular portion converging towards the lower end of the inner tubular wall thereby presenting a smooth inner peripheral surface to said cylindrical container wall and permitting said lid to be slidably mounted and dismounted from said container, and the other of said stable states being defined by said frusto-conical annular portion diverging away from the lower end of the inner tubular wall thereby deforming said inner tubular wall adjacent said lower end to create an outwardly directed peripheral locking bead which, when said lid is mounted on the container, engages and grips said inner surface of said cylindrical container wall.

The present invention further provides a container in combination with a removable closure as described hereinabove, the cylindrical container wall of such closure having a peripheral groove in at least its outer surface adjacent the frusto-conical wall portion to provide a seal for said inwardly facing locking bead.

An embodiment of the present invention will now be described by way of example only with reference to the accompanying drawings, in which:

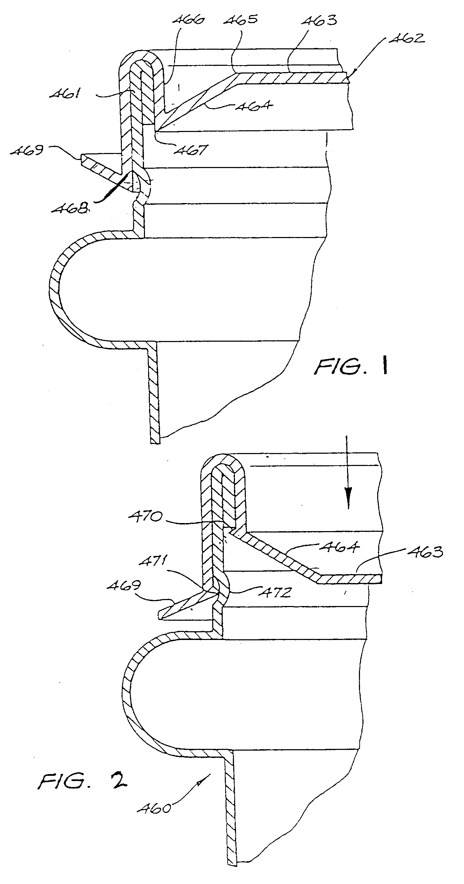

FIGS. 1 and 2 show partial cross-sections of an insert for an open mouthed container, in unlocked and locked positions.

Figures 1 and 2 show a part view of a lid according to the invention for an open mouthed container.

The container 460 has a cylindrical wall 461 which defines an opening. The lid 462 comprises a disc 463 having a frusto-conical wall 464 depending from its outer circumference 465. The wall 464 in turn has a hollow U-shaped ring 466 at its outer circumference 467. The ring 466 is downwardly open and of a diameter to allow the wall 461 to enter it. From the outer end 468 of the ring 466 is a second frusto-conical wall 469. In the as-formed and undeformed state of Figure 1 the wall 464 is upwardly converging while wall 469 is downwardly converging. In the state of Figure 1 the lid is placed over the container to engage wall 461 and then walls 463, 464 and 469 are downwardly depressed such that walls 464 and 469 reverse their orientation, as in Figure 2. This reversal of orientation causes ring shaped beads 470 and 471 to form on the U-shaped ring 466 which engage the wall 468, sealingly locking the lid 460 to the container.

Preferably, but not essentially, the wall 468 has an annular groove 472 into which bead 471 may engage so as to enhance the security of the lid. A further preferred feature is a large radially outwardly extending ring 473 which protects the wall 469 from accidental release.

It is obvious that many adaptations and modifications may be made to the invention without departing from the scope of the invention.

## Claims

1. A removable closure for a container (460) having a cylindrical container wall (461) with an inner and an outer surface defining an opening, said closure comprising a lid (462) for cooperating with the cylindrical container wall (461) to close said opening, said lid (462) comprising a disc portion (463) extending across said opening and a cylindrical tubular wall extending along the outer surface of said cylindrical container wall (461) in closely spaced relation when said lid (462) is mounted telescopically on said container (460); characterised in that said tubular wall has a lower end with a frusto-conical wall portion (469) extending therefrom circumferentially about the periphery of said tubular wall, said frusto-conical wall portion (469) being displaced between either of two stable states, one of said stable states being defined by said frusto-conical wall portion (469) converging towards the lower end of the tubular wall thereby presenting a smooth inner peripheral surface to said cylindrical container wall (461) and permitting said lid (462) to be slidably mounted and dismounted from said container (460), and the other of said stable states being defined by said frusto-conical wall portion (469) diverging away from the lower end of the tubular wall thereby deforming said tubular wall adjacent said lower end to create an inwardly facing peripheral locking bead (471) which, (461) when the lid (462) is mounted on the container (460), engages and grips said outer surface of said cylindrical container wall thereby preventing removal of the lid (462) from the container (460).

2. A removable closure for a container (460) as defined in claim 1, wherein the lid (462) further comprises an inner tubular wall engageable within the container opening, said disc portion (463) comprising a frusto-conical annular portion (464) connected to said inner tubular wall at a lower end of the inner tubular wall, said frusto-conical annular portion (464) being displaceable between either of two stable states, one of said stable states being defined by said frusto-conical annular portion (464) converging towards the lower end of the inner tubular wall thereby presenting a smooth inner peripheral surface to said cylindrical container wall (461) and permitting said lid (462) to be slidably mounted and dismounted from said container (460), and the other of said stable states being defined by said frusto-conical annular portion (464) diverging away from the lower end of the inner tubular wall thereby deforming said inner tubular wall adjacent said lower end to create an outwardly facing peripheral locking bead (470) which, when said lid (462) is mounted on the container (460), engages and grips said inner surface of said cylindrical container wall (461).

3. A container (460) in combination with a removable closure as defined in claim 1 or claim 2, wherein the cylindrical container wall (461) has a peripheral groove (472) in at least its outer surface adjacent the frusto-conical wall portion (469) to provide a seat for the inwardly facing locking bead (471).

## Patentansprüche

1. Abnehmbarer Verschluß für einen Behälter (460), der eine zylindrische Behälterwand (461) mit einer Innen- und einer Außenfläche aufweist und eine Öffnung bildet, wobei der Verschluß aus einem Deckel (462) besteht, der mit der zylindrischen Behälterwand (461) zum Verschließen der Öffnung zusammenwirkt, wobei der Deckel (462) einen Scheibenteil (463), der sich quer zur Öffnung erstreckt, und eine zylindrische rohrförmige Wand aufweist, die sich längs der Außenfläche der zylindrischen Behälterwand (461) in eng benachbarter Lage erstreckt, wenn der Deckel (462) teleskopisch auf den Behälter (460) aufgeschoben ist; dadurch gekennzeichnet, daß die rohrförmige Wand ein unteres Ende mit einem kegelstumpfförmigen Wandabschnitt (469) aufweist, der sich von dieser in Umfangsrichtung um den Umfang der rohrförmigen Wand erstreckt, wobei der kegelstumpfförmige Wandabschnitt (469) zwischen zwei stabilen Zuständen verschiebbar ist, wobei einer der stabilen Zustände darin besteht, daß der kegelstumpfförmige Wandabschnitt (469) gegen das untere Ende der rohrförmigen Wand konvergiert und dadurch der zylindrischen Behälterwand (461) eine glatte innere Umfangsfläche darbietet und ein gleitbares Anbringen und Abnehmen des Deckels (462) vom Behälter (460) gestattet, und der andere stabile Zustand darin besteht, daß der kegelstumpfförmige Wandabschnitt (469) vom unteren Ende der rohrförmigen Wand weg divergiert und dadurch die rohrförmige Wand nahe dem unteren Ende verformt, um einen nach innen weisenden, um den Umfang verlaufenden Sperrwulst (471) zu erzeugen, der, wenn der Deckel (462) auf den Behälter (460) aufgesetzt ist, an der Behälterwand (461) angreift und die Außenfläche der zylindrischen Behälterwand ergreift, wodurch ein Abnehmen des Deckels (462) vom Behälter (460) verhindert wird.

2. Abnehmbarer Verschluß für einen Behälter (460) nach Anspruch 1, bei welchem der Deckel (462) eine innere rohrförmige Wand aufweist, die innerhalb der Behälteröffnung eingreifen kann, wobei der Scheibenteil (463) einen kegelstumpfförmigen Ringabschnitt (464) aufweist, der mit der inneren rohrförmigen Wand an einem unteren Ende der inneren rohrförmigen Wand verbunden ist, der kegelstumpfförmige Ringabschnitt (464) zwischen zwei stabilen Zuständen verschiebbar ist, der eine stabile Zustand darin besteht, daß der kegelstumpfförmige Ringabschnitt (464) zum unteren Ende der inneren rohrförmigen Wand hin konvergiert und dadurch eine glatte innere Umfangsfläche der zylindrischen Behälterwand (461) darbietet und ein gleitbares Aufsetzen und Abnehmen des Deckels (462) auf den bzw. von dem Behälter (460) gestattet, und wobei der andere stabile Zustand darin besteht, daß der kegelstumpfförmige Ringabschnitt (464) vom unteren Ende der inneren rohrförmigen Wand weg divergiert und dadurch die innere rohrförmige Wand nahe dem unteren Ende verformt, so daß sie einen nach außen weisenden, um den Umfang verlaufenden Sperrwulst (470) bildet, der, wenn der Deckel (462) auf den Behälter (460) aufgesetzt ist, an der Innenfläche der zylindrischen Behälterwand (461) angreift und diese ergreift.

3. Behälter (460) in Kombination mit einem abnehmbaren Verschluß nach Anspruch 1 oder 2, bei welchem die zylindrische Behälterwand (461) eine Umfangswelle (472) wenigstens in ihrer Außenfläche nahe dem kegelstumpfförmigen Wandabschnitt (469) aufweist, um einen Sitz für den nach innen weisenden Sperrwulst (471) zu bilden.

## Revendications

1. Fermeture amovible pour un récipient (460) ayant une paroi cylindrique (461) avec des surfaces interne et externe définissant une ouverture, cette fermeture comprenant un couvercle (462) pour coopérer avec la paroi cylindrique (461) du récipient pour fermer l'ouverture, le couvercle (462) comprenant une partie en forme de disque (463) s'étendant en travers de l'ouverture et une paroi tubulaire cylindrique s'étendant le long de la surface externe de la paroi cylindrique (461) du récipient de façon proche de celle-ci quand le couvercle (462) est monté téléscopiquement sur le récipient (460) ; caractérisée en ce que la paroi tubulaire a une extrémité inférieure munie d'une partie de paroi tronconique (469) s'étendant à partir de là circonférentiellement par rapport à la périphérie de la paroi tubulaire, la partie de paroi tronconique (469) étant déplaçable entre deux états stables, l'un des états stables état défini par le fait que la partie de paroi tronconique (469) converge vers l'extrémité inférieure de la paroi tubulaire présentant ainsi une surface périphérique interne lisse vers la paroi cylindrique (461) du récipient et permettant au couvercle (462) d'être monté à coulissement et démonté par rapport au récipient (460), et l'autre état stable étant défini par le fait que la partie de paroi tronconique (469) diverge par rapport à l'extrémité inférieure de la paroi tubulaire déformant ainsi la paroi tubulaire au voisinage de l'extrémité inférieure pour créer une arête de verrouillage périphérique (471) tournée vers l'intérieur qui, quand le couvercle (462) est monté sur un récipient (460), entre en contact avec et frotte la surface externe de la paroi cylindrique (461) du récipient empêchant ainsi d'enlever le couvercle (462) du récipient (460).

2. Fermeture amovible pour un récipient (460) selon la revendication 1, dans laquelle le couvercle (462) comprend en outre une paroi tubulaire interne qui peut rentrer dans l'ouverture du récipient, la partie de disque (463) comprenant une partie annulaire tronconique (464) reliée à la paroi tubulaire interne au niveau d'une extrémité inférieure de la paroi tubulaire, la partie annulaire tronconique (464) étant déplaçable entre deux états stables, l'un des états stables étant défini par le fait que la partie annulaire tronconique (464) converge vers l'extrémité inférieure de la paroi tubulaire interne présentant ainsi une surface périphérique interne lisse à la paroi cylindrique (461) du récipient et permettant au couvercle (462) d'être monté et démonté à coulissement par rapport au récipient (460), et l'autre des états stables étant défini par le fait que la partie annulaire tronconique (464) diverge par rapport à l'extrémité inférieure de la paroi tubulaire interne déformant ainsi la paroi tubulaire interne au voisinage de son extrémité inférieure pour créer une arête de fermeture périphérique tournée vers l'extérieur (470) qui, quand le couvercle (462) est montée sur le récipient (460), entre en contact avec et frotte contre la surface interne de la paroi cylindrique (461) du récipient.

3. Récipient (460), en combinaison avec une fermeture amovible selon la revendication 1 ou la revendication 2, dans lequel la paroi cylindrique (461) du récipient comprend une rainure périphérique (472) dans au moins sa surface externe voisine de la partie de paroi tronconique (469) pour assurer un appui à l'arête de fermeture tournée, vers l'intérieur (471).
